(19) Europäisches Patentamt
European Patent Office
Office européen des brevets

(11) Publication number : 0 453 247 A2

# (12) EUROPEAN PATENT APPLICATION

(21) Application number : 91303395.7

(22) Date of filing : 17.04.91

(51) Int. Cl.$^5$ : C07H 19/06, A61K 31/70

(30) Priority : 18.04.90 GB 9008696

(43) Date of publication of application : 23.10.91 Bulletin 91/43

(84) Designated Contracting States : AT BE CH DE DK ES FR GB GR IT LI LU NL SE

(71) Applicant : THE WELLCOME FOUNDATION LIMITED
Unicorn House 160 Euston Road
London NW1 2BP (GB)

(72) Inventor : Slater, Martin John
The Wellcome Research Laboratories
Langley Court, Beckenham, Kent BR3 3BS (GB)

(74) Representative : Stott, Michael John et al
The Wellcome Research Laboratories Group
Patents & Agreements Langley Court
Beckenham Kent BR3 3BS (GB)

## (54) Anti-viral compounds.

(57) Fluoro-substituted pyrimidine nucleosides are disclosed which are useful in the treatment or prophylaxis of herpes virus infections.

EP 0 453 247 A2

The present invention relates to 2′-deoxy-2′-fluoro-5-alkynyl pyrimidine nucleosides, and derivatives thereof, to methods for their preparation, to compositions containing them and to their use in therapy particularly for the treatment of certain viral infections.

Of the DNA viruses, the herpes group is the source of the most common viral illnesses in man. The group consists of the herpes simplex viruses (HSV-1 and HSV-2), cytomegalovirus (CMV), varicella-zoster virus (VZV), Epstein-Barr virus (EBV) and human herpes virus 6 (HHV-6).

HSV-1 and HSV-2 are two of the most common infectious agents of man. These viruses are able to persist in the host's neural cells; once infected, individuals are at risk of recurrent clinical manifestations of infection which can be both physically and psychologically distressing. Clinical conditions of HSV infection include extensive and debilitating lesions of the skin, mouth and/or genitals. Primary infections may be subclinical although they tend to be more severe than infections in individuals previously exposed to the virus. Ocular infection by HSV can lead to keratitis or cataracts thereby endangering the host's sight. Infection in the newborn, in immunocompromised patients or penetration of the infection into the central nervous system, can prove fatal.

Transmission of the virus is by direct physical contact between a host and a recipient; the spread of HSV infection is therefore considered a very significant social problem, particularly as no effective vaccine is available.

Varicella zoster virus (VZV) is a herpes virus which causes chicken-pox and shingles. Chicken-pox is the primary disease produced in a host without immunity and in young children is usually a mild illness characterised by a vesicular rash and fever. Shingles or zoster is the recurrent form of the disease which occurs in adults who were previous infected with varicella-zoster virus. The clinical manifestions of shingles are characterised by neuralgia and a vesicular skin rash that is unilateral and dermatomal in distribution. Spread of inflammation may lead to paralysis or convulsions. Coma can occur if the meninges become affected. In immunodeficient patients VZV may disseminate causing serious or even fatal illness. VZV is of serious concern in patients receiving immunosuppressive drugs for transplant purposes or treatment of malignant neoplasia and is a serious complication of AIDS patients due to their impaired immune system.

Epstein-Barr virus (EBV) causes infectious mononucleosis, and is also suggested as the causative agent of nasopharyngeal cancer, immunoblastic lymphoma,Burkitt's lymphoma and hairy leukoplakia.

HHV6 is the causative agent of rosela infantum (exanthum subitum) in children which is characterised by fever and the appearance of a rash after the fever has declined.

Certain fluorinated nucleosides have been disclosed as having antiparasitic activity. For example, EP-A-O 287 313 discloses 2′-fluoro-2′3′-dideoxynucleosides. EP-A-O 219 829 discloses 2′-deoxy-2′-fluoro-β- D-arabinofuranosyl nucleosides which are described as having antiparasitic activity, especially against Leishmania.

EPA-0-272 065 describes certain pyrimidine nucleosides characterised by the presence of an unsaturated grouping in the 5-position, which are of particular value in medical therapy particularly for the treatment of certain viral infections including VZV.

The Applicants have now identified a class of fluoro-substituted pyrimidine nucleosides which may be used in the treatment or prophylaxis of herpes virus infections, for example, varicella zoster virus (VZV), herpes simplex virus (HSV) especially HSV-1, Epstein-Barr virus (EBV) and Human Herpes virus-6 (HHV-6) in mammals including man.

According to the present invention therefore there are provided compounds of formula (I):

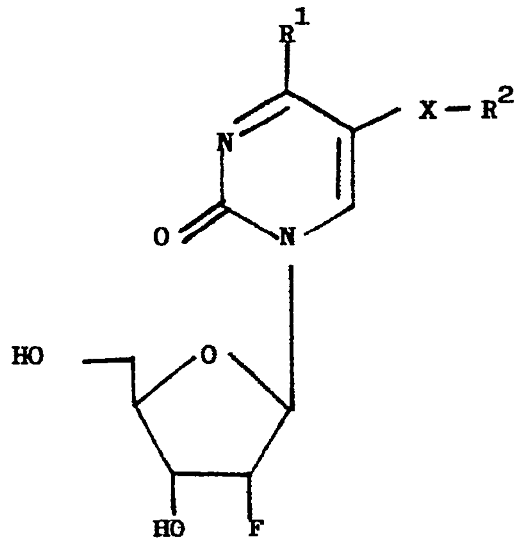

(I)

wherein $R^1$ represents a hydroxy or amino group;
X represents a $C_{2-4}$ alkynylene group, a $C_{2-4}$ alkenylene group optionally substituted by one or more halogen radicals, or a $C_{1-4}$ alkylene group optionally substituted by one or more halogen radicals;
and $R^2$ represents a hydrogen atom, a $C_{1-2}$ alkyl group, a $C_{3-4}$ branched alkyl group eg. isopropyl, a $C_{3-4}$ cycloalkyl group, or a $C_{3-6}$ cycloalkyl-$C_{1-6}$ alkyl group eg. cyclopropylmethyl; with the proviso that the compound of formula (I) is not 5-vinyl-2′-fluoro-2′-deoxyuridine; and the physiologically acceptable salts, esters and salts of esters thereof;

The invention also includes bioprecursors or "pro-drugs" of the above-defined compounds, that is compounds which are converted in vivo to compounds of formula (I) or physiologically acceptable salts or esters thereof.

Preferred esters of the compounds of formula (I) include carboxylic acid esters in which the non-carbonyl moiety of the ester grouping is selected from straight or branched chain alkyl (e.g. methyl, n-propyl, n-butyl or t-butyl), alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy or amino); sulphonate esters such as alkyl- or alkylarylsulphonyl (e.g. methanesulphonyl); amino acid esters (e.g. L-valyl or L-isoleucyl); and mono-, di- or tri-phosphate esters. In such esters unless otherwise specified, any alkyl moiety present advantageously contains 1 to 18 carbon atoms, particularly 1 to 4 carbon atoms. Any aryl moiety present in such esters advantageously comprises a phenyl group. Any reference to any of the above compounds also includes a reference to a physiologically acceptable salt thereof.

Examples of physiologically acceptable salts of the compound of formula (I) and physiologically acceptable esters thereof include base salts, e.g. derived from an appropriate base, such as alkali metal (e.g. sodium), alkaline earth metal (e.g. magnesium) salts, ammonium and $NX_4^+$ (wherein X is $C_{1-4}$ alkyl). Physiologically acceptable salts formed from a hydrogen atom or an amino group from compounds of formula (I) include salts of organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids. Physiologically acceptable salts of a compound of an hydroxy group include the anion of said compound in combination with a suitable cation such as $Na^+$, $NH_4^+$, and $NX_4^+$ (wherein X is a $C_{1-4}$ alkyl group).

The pyrimidine nucleosides of formula (I) and physiologically acceptable salts, esters and salts of esters thereof will be hereinafter referred to as the compounds according to the invention.

It will be appreciated that when $R^1$ represents a hydroxy group then formula (I) can be in its corresponding tautomeric form.

Preferred compounds of formula (I) include those wherein

(a) $R^1$ represents a hydroxy or amino group; or

(b) X represents a $C_1$ alkynyline group; or

(c) $R^2$ represents a hydrogen atom or a methyl group.

The following compounds of formula (I) are most preferred compounds according to the invention particularly by virtue of their especially potent antiviral activity against VZV :-

(f) 2′-deoxy-2′-fluoro-5-prop-1-ynyluridine
(g) 2′-deoxy-5-ethynyl-2′-fluorocytidine
(h) 2′-deoxy-2′-fluoro-5-prop-1-ynylcytidine
(i) 2′-deoxy-5-ethynyl-2′-fluorouridine

The present invention further includes:

(a) compounds of formula (I) including 5-vinyl-2′-fluoro-2′-deoxyuridine for use in medical therapy;
(b) pharmaceutical formulations of compounds according to the invention with one or more acceptable carriers therefor and optionally other therapeutic ingredients;
(c) a method for the treatment or prophylaxis of a herpes (e.g. VZV or HSV) viral infection which comprises treating a subject with an effective amount of a compound according to the invention;
(d) use of a compound according to the invention in the manufacture of a medicament for use in the treatment or prophylaxis of a herpes (e.g. VZV or HSV) viral infection;
(e) a process as described below for the preparation of compounds according to the invention.

In a further aspect of the present invention there is provided any and all novel intermediates disclosed herein.

The compounds according to the invention may be employed for the treatment or prophylaxis of clinical conditions associated with herpes viral infections, (e.g. herpes keratitis, herpes labialis or herpes genitalis (caused by HSV-I or 2) or chicken pox or shingles (caused by VZV).

The compounds according to the invention may be administered by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with, for example, the condition of the recipient.

For each of the above-indicated utilities and indications the amount required of the individual active ingredients will depend upon a number of factors including the severity of the condition to be treated and the identity of the recipient and will ultimately be at the discretion of the attendant physician. In general, however, for each of these utilities and indications, a suitable, effective dose will be in the range 0.1 to 250 mg per kilogram body weight of recipient per day, preferably in the range 1 to 100 mg per kilogram body weight per day and most preferably in the range 5 to 30 mg per kilogram body weight per day; an optimum dose is about 15 mg per kilogram body weight per day (unless otherwise indicated all weights of active ingredient are calculated as the parent compound; for salts and esters thereof the figures would be increased proportionately.) The desired dose may if desired be presented as two, three, four or more sub-doses administered at appropriate intervals throughout the day. These sub-doses may be administered in unit dosage forms, for example, containing 10 to 1000 mg. preferably 20 to 500 mg and most preferably 100 to 400 mg of active ingredient per unit dosage form.

While it is possible for the compounds to be administered alone it is preferable to present them as pharmaceutical formulations. The formulations of the present invention comprise at least one active ingredient, as above defined, together with one or more acceptable carriers thereof and optionally other therapeutic ingredients. The carrier(s) must be "acceptable" in the sense of being compatible with the other ingredients of the formulation and not deleterious to the recipients thereof.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid, or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose

in varying proportions to provide desired release profile.

For infections of the eye or other external tissues, e.g. mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, i.e. an alcohol having two or more hydroxyl groups such as propylene glycol, butane-1,3-diol, mannitol, sorbitol glycerol and polyethylene glycol and mixtures thereof. The topical formulations may desirably include a compound which ehances absorption or penetration of the active ingredient through the skin or other affected areas. Examples of such dermal penetration ehancers include dimethylsulphoxide and related analogues.

The oily phase of the emulsions of this invention may be constituted from known ingredients in a known manner. While this phase may comprise merely an emulsifier (otherwise known as an emulgent), it desirably comprises a mixture of at least one emulsifier with a fat or an oil or with both a fat and an oil.

Preferably, a hydrophilic emulsifier is included together with a lipophilic emulsifier which acts as a stabilizer. It is also preferred to include both an oil and a fat. Together with emulsifier(s) with or without stabilizer(s) make up the so-called emulsifying wax, and the wax together with the oil and/or fat make up the so-called emulsifying ointment base which forms the oily dispersed phase of the cream formulations.

Emulgents and emulsion stabilizers suitable for use in the formulation of the present invention include Tween 60, Span 80, cetostearyl alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono-or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

It should be understood that in addition to the ingredients particularly mentioned above the formulations

of this invention may include other agents conventional in the art having regard to the type of formulation in question, for example those suitable for oral administration may include flavouring agents.

The compounds according to the invention may be prepared by any of the methods known in the art for the preparation of similar compounds e.g. see UK Patent Specification No. 1 601 020, or Robins M.J., and Barr, P.J., J.Org.Chem. (1983) 43, 1854-1862, and in particular the processes desribed in the Examples given hereinafter.

The present invention also provides a process for the preparation of a compound according to the invention which comprises;

A. condensing a compound of formula

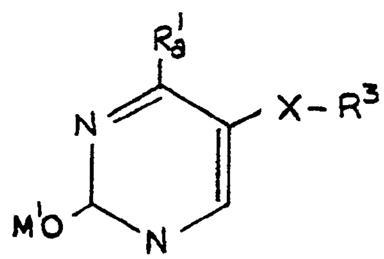

(II)

(wherein $R^3$ is a protecting group which can be converted to H (eg. $SiR^4_3$ wherein $R^4$ is a $C_{1-4}$ alkyl group) or is defined as for $R^2$, X is as defined above (p.3) $R^1_a$ represents a protected hydroxy or amino group and $M^1$ represents a hydroxy protecting group) with a compound of formula (III) (Coddington, J.F., et al., Carbohyd.Res., (1966) 1, 455).

(III)

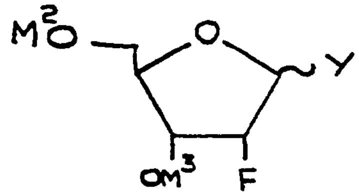

(wherein Y represents a halogen atom or acyl group (eg. -CO-$R^5$ wherein $R^5$ is $C_{1-4}$ alkyl) or alkyl group (e.g. $OR^5$) and $M^2$ and $M^3$ each represent a hydroxy-protecting group to form a compound of formula (IIIa); or

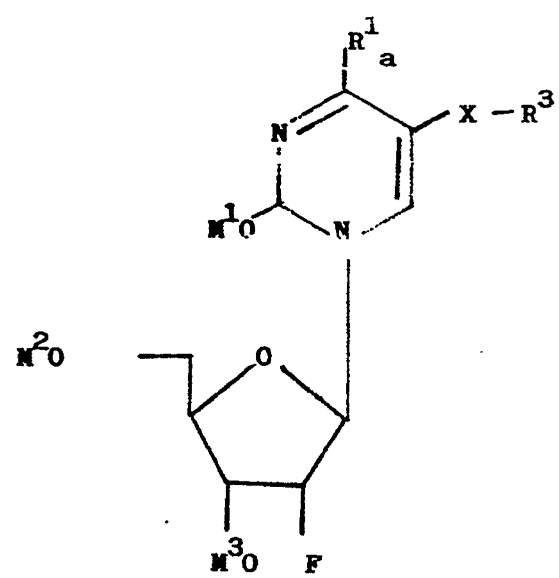

(IIIa)

B. reacting a compound of formula (IV)

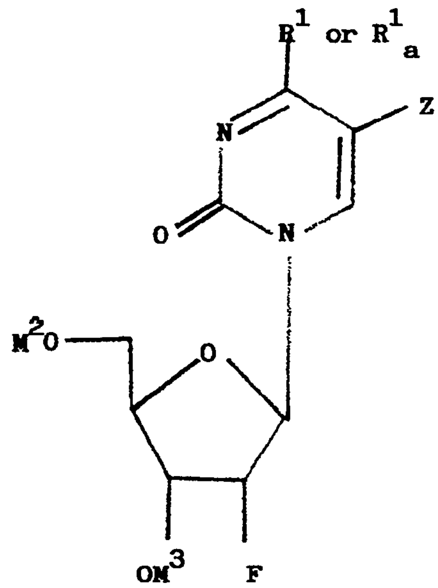

(IV)

wherein $R^1$ and $R^1_a$, $M^2$ and $M^3$ are as defined above and Z is a leaving group e.g. iodide, (Kumar, R., et al., J.Med.Chem., (1990) 33, 317), with a compound capable of providing the necessary grouping of formula -X-$R^3$ (in which X is ethynylene and $R^3$ is as defined above); or

C. reacting a compound of formula (V)

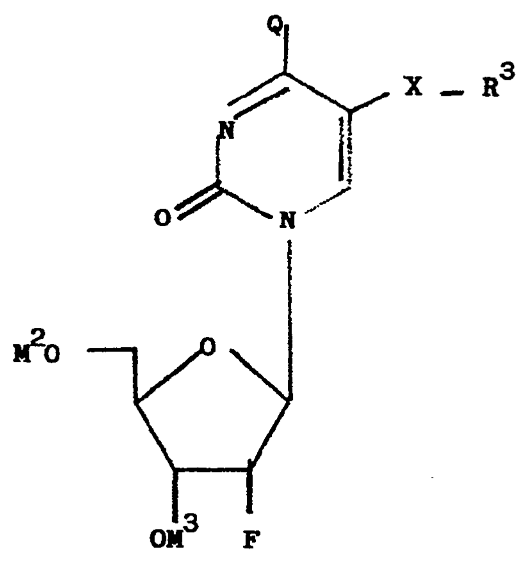

(V)

(wherein Q is an appropriate leaving group and X,$R^3$,$M^2$ and $M^3$ are as defined above) with an agent serving to replace the group Q with an amino group to form a compound of formula (I) in which $R^1$ is amino and $R^3$ is as described above; or

D. decarboxylative halogenation (e.g. using an N-halosuccinimide) of a compound of formula (VI) (Kumar, R., et al., J.Med.Chem., (1990) 33, 717) below

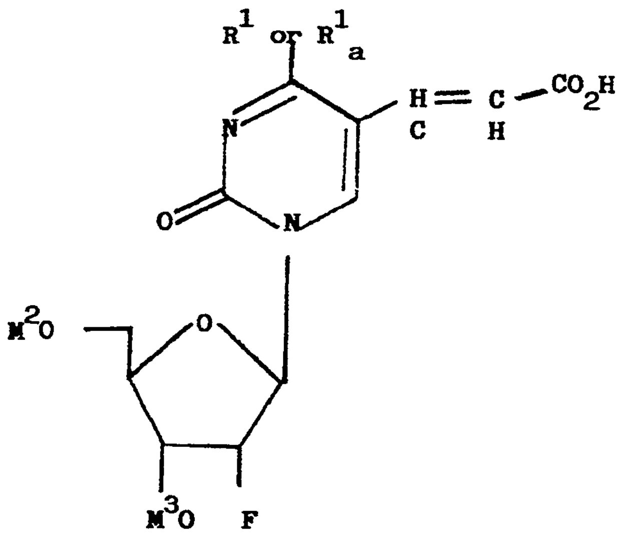

(VI)

wherein $R^1$, $R^1_a$, $M^2$ and $M^3$ are as defined above to form a compound of formula (I) in which X is ethenylene substituted with a halogen atom and $R^2$ is hydrogen; or

E. halogenating a compound of formula (VII) (Kumar, R., et al., J.Med.Chem., (1990) 33, 717) below

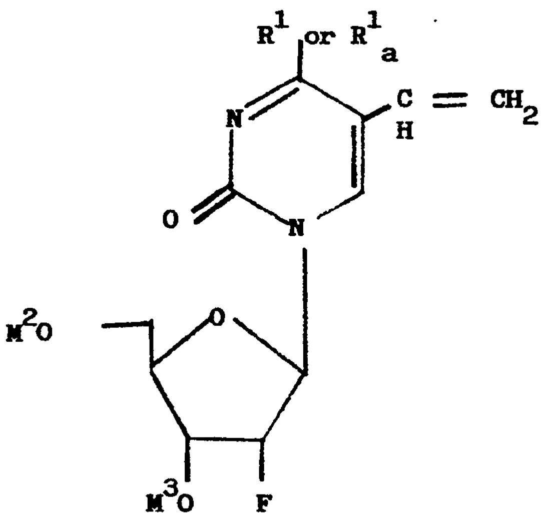

(VII)

wherein $R^1$, $R^1_a$, $M^2$ and $M^3$ are as defined above to form a compound of formula (I), in which X is ethenylene substituted with a halogen atom and $R^2$ is a hydrogen atom; or

F. reacting a compound of formula (VIII)

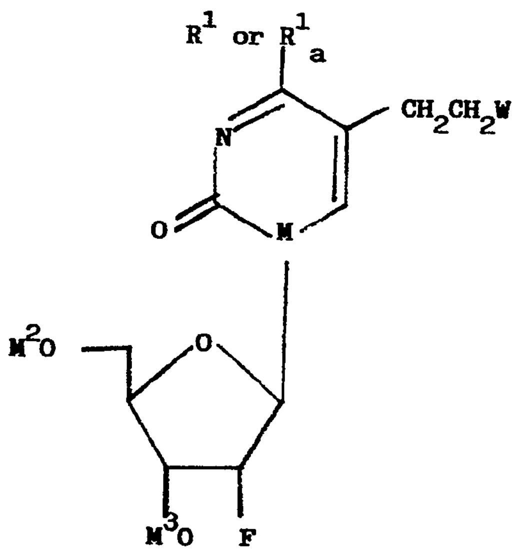

(VIII)

(wherein W is a leaving group) under conditions serving to replace the said leaving group by a halogen atom to form a compound of formula I wherein X is a halogen-substituted alkylene group and $R^2$ is hydrogen;
G. Reacting a compound of formula (IX)

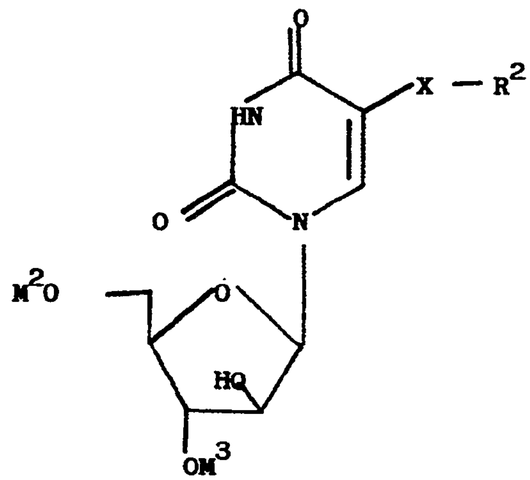

(IX)

(wherein X, $R^2$, $M^2$ and $M^3$ are as defined above) with a dialkylamino sulphur trifluoride e.g. diethylamino sulphur trifluoride or other suitable derivative of $SF_4$ to introduce a fluorine radical at the 2'-position of the sugar moiety, to form a compound of formula (IIIa) in which $R^1$ is hydroxy.
H. Reacting a compound of formula (X)

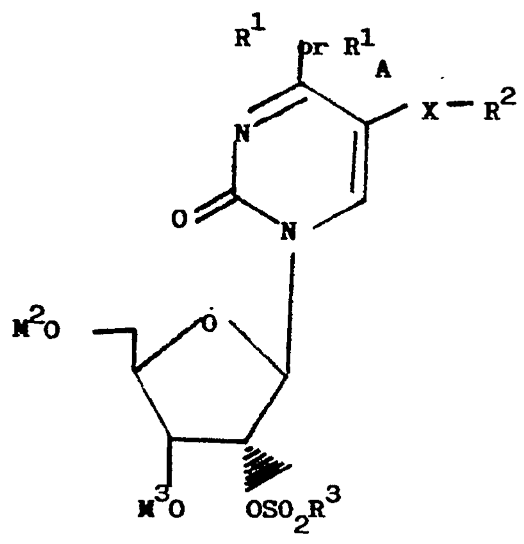

(X)

(wherein X, $R^2$, $R^3$, $M^2$ and $M^3$ are as defined above) with a source of fluoride ions such as potassium fluoride or tetrabutylammonium fluoride, advantageously in a suitable solvent such as dimethylformamide or tetrahydrofuran, to introduce a fluorine radical at the 2'-position of the sugar moiety, to form a compound of formula (IIIa).

and optionally thereafter or simultaneously therewith, performing either or both of the following, in any desired order:

(i) removing any remaining protecting groups

(ii) where the resulting compound is a compound of formula (I), converting it into a physiologically acceptable salt, ester or salt of ester thereof or, where the resulting compound is a physiologically acceptable salt or ester, converting it into a different physiologically acceptable salt, ester or salt of ester or a compound of formula (I).

With regard to process A, the starting materials may be protected with conventional protecting groups such as acyl groups, e.g. alkanoyl or aroyl groups such as p-toluoyl, or trialkylsilyl groups such as the trimethylsillyl group, the $M^1$ and $R^1_a$ protecting groups being generally silyl protecting groups. The reaction between compounds of formulae (II) and (III), may be carried out in the presence of a Lewis acid catalyst, for example stannic chloride or trimethylsilyl triflate, in an appropriate solvent such as acetonitrile or 1,2-dichloroethane. The parent compound can then be obtained, following anomeric separation, by treatment with alcoholic base, such as sodium methoxide in methanol. The process is also described by Barr, et al., in J.Chem.Soc., Perkin Trans 1 (1978), 1263 et seq. The protecting groups can subsequently be removed by acid or base hydrolysis, hydrogenolysis or fluoride, acyl groups being advantageously removed by base hydrolysis.

Regarding process B, this can be carried out using analogous procedures to those described by Robins, M.J., and Barr, P.J., in J.Org.Chem. (1983) 48, 1854 et seq. A 5-halogenated nucleoside such as 2'-deoxy-5-iodouridine in a suitably protected form, for example with the protecting groups referred to above, can be reacted in the presence of a catalyst e.g. a palladium catalyst, with an appropriate alkyne, such as trimethylsilylacetylene, in the presence of an organic base, such as triethylamine, and another metal catalyst, for example a copper (1) salt, at an elevated temperature such as 50°C to give the protected acetylenic nucleoside. A preferred palladium catalyst is bis(triphenylphosphine)-palladium dichloride and a preferred copper catalyst is cuprous iodide. The parent compound can readily be obtained by removal of any protected groups for example by treatment with alcoholic base, such as sodium methoxide in methanol.

In process B, the compound of formula (IV) particularly wherein Z represents a halogen atom such as iodine and $R^4$ represents a protected hydroxy group can be prepared for example by methods analogous to those described by Schinazi et al, J.Med.Chem. 1979. 22(20) 1273.

In process C, the leaving group Q is advantageously a suitable heterocyclyl group advantageously a 1,2,4-triazol-1-yl group or a halo e.g. chloro group, the removal of the group Q being suitably effected by treatment of the compound of formula (V) with ammonia.

The compound of formula (V) may be prepared for example by treating the corresponding 4-oxo compound with an appropriate functionalising agent serving to introduce the leaving group Q for example by treatment with 1,2,4-triazole, conveniently in the presence of a condensing agent such as 4-chlorophenylphosphodichloridate, e.g. in a base solvent advantageously pyridine or by treatment of the 4-oxo compound with thionyl chloride in dimethylformamide.

Regarding process D, a compound of formula (VI) can be prepared from hydrolysis ester (E)-5-(2-carboalkoxyvinyl)-2'-deoxy-2'-fluorouridine. The starting carboalkoxyvinyl compound can be prepared from a compound of formula (IV) by a Heck reaction with e.g. vinyl carboxylate in the presence of a catalyst e.g. a palladium catalyst (Tetrahedron, Vol. 43, No. 20, 4601-4608 (1987)).

With reference to process E, a compound of formula (I) can be prepared from a 5-vinyl-2'-fluoro-2'-deoxyuridine compound by a halogenation reaction using for example $Br_2$ (J.Med.Chem., 33, 717 (1990)). The starting 5-vinyl compound can be prepared from a compound of formula (VI) in a decarboxylative halogenation reaction as described in Jones, A.S., et al, JCS, Perkin, 1, 1325, (1987).

In process F a compound of formula (VIII) wherein W is OH may be reacted with a halogenating reagent such as a tetrahalomethane compound (eg. $CC1_4$) and triphenylphosphine whereby W is replaced by a halogen radical, or by reacting a compound of formula (VIII) where W is $OSO_2R$ with a halide salt (eg. LiCl, $Bu_4NBr$) in a suitable solvent such as DMF whereby W is replaced by a halogen radical, or where W is already a halogen atom by carrying out a halide exchange reaction (for example replacing idodide by chloride), to form a compound of formula (IIIa).

In process G, compounds of formula (IX) may be prepared by selective protection of the 5'- and 3'- hydroxyl groups with, for example, triphenylmethyl chloride. Compounds of formula (IX) may be prepared by the process described by Bobek et al. in J.Med.Chem., (1987), 30, 2154.

With reference to process H, compounds of formula (X) can be prepared from compounds of formula (IX) by treatment with a sulphonyl halide (e.g. trifluoromethanesulfonyl chloride) in the presence of a base (e.g. pyridine, NaH).

The above-mentioned starting materials may be prepared in conventional manner from known compounds using techniques that are known in the art for example as described in Nucleic Acid Chemistry: Improved New Synthetic Procedures, Methods and Techniques. Ed.L.B. Townsend and R.S. Tipson. Wiley Walker, E de Clercq and F. Eckstein. NATO Advanced Study Institute, Plenum Press (1979), Examples of methods for the preparation of the starting materials are described below.

The compounds of formula (I) in which X represents a vinylene group may be prepared from corresponding compounds in which X represents an ethynylene group for example by catalytic hydrogenation using an appropriate catalyst for example a Lindlar catalyst poisoned with quinoline, eg. in an alcoholic solvent such as methanol or ethanol. Other methods for preparing these vinylene compounds are described by example by S.G. Rahim et al Nucleic Acids Research 1982 10(17), 5285.

Esters according to the invention may be prepared in conventional manner eg. by treatment of the parent compound of formula (I) or an ester thereof (optionally protected) with an appropriate esterifying or transesterifying agent respectively, for example, by treatment of 2'-deoxy-5-ethynyluridine with an appropriate acid halide (eg. chloride) or anhydride in the presence of base, conveniently pyridine, which may also be used as the solvent, any remaining protecting groups being thereafter removed.

Salts according to the invention may also be prepared in conventional manner for example by reaction of the parent compound with an appropriate base to form the corresponding base salt. Other derivatives according to the invention can also be prepared in conventional manner.

The following Examples are intended for illustration only and are not intended to limit the scope of the invention in any way.

## Example 1

2'-Deoxy-5-ethynyl-2'-fluoro uridine

To a solution of 2'-deoxy-2'-fluoro-5-iodouridine (810mg, 2.18mmol) in pyridine (10ml) at 0°C was added p-toluoyl chloride (0.6ml) with stirring. After 30 minutes the mixture was stirred at room temperature for 5 hours. The mixture was then evaporated in vacuo, co-evaporated with ethanol and toluene and purified by flash chromatography over $SiO_2$, eluting with $CHCl_3$, followed by $CHCl_3$/MeOH (20:1), then (10:1). The crude product (containing starting material) was collected and re-processed with pyridine (10ml) and p-toluoyl chloride (0.06ml) as described above. Flash chromatography over $SiO_2$, eluting with toluene/EtOAc (6:1), then (4:1), (3:1), and finally (1:1) afforded 2'-deoxy-3',5'-di-O-p-toluoyl-5-iodo-2'-fluorouridine.

A solution of 2'-deoxy-3',5'-di-O-p-toluoyl-5'-iodo-2'-fluorouridine (740mg, 1.21mmol) in N-ethylmorpholine (18ml) was purged with oxygen-free dry $N_2$. CuI (29mg), Pd $Cl_2(PPh_3)_2$ (31.5mg) and trimethylsilylacetylene (260mg) were then added and the solution stirred at room temperature. After four hours a further 100mg of trimethylsilylacetylene was added, and stirring continued at room temperature overnight. The mixture was then filtered, the precipitate washed with ether, and the filtrate evaporated. The residue was dissolved in $CHCl_3$ (50ml), washed with 5% EDTA solution (2x40ml) and water (40ml). The organic fraction was dried ($Na_2SO_4$),

evaporated, and purified by flash chromatography ($SiO_2$), eluting with toluene/EtOAc (4:1), then (3:1). The fractions containing the product were evaporated to afford 2′-deoxy-3′,5′-di-O-p-toluoyl-2′-fluoro-5-(2-trimethylsilyl)-ethynyluridine. This compound (510mg, 0.88mmol) was deprotected with sodium methoxide (3eq) in MeOH (30ml) at room temperature. The solution was neutralised with Dowex ($H^+$) resin and filtered. The fitrate was evaporated and purified by flash chromatography over $SiO_2$, eluting with $CHCl_3$/MeOH (15:1), (10:1), (6:1), and finally (4:1). Crystallisation from hot MeOH provided 2′-deoxy-5-ethynyl-2′-fluorouridine as a hemi-hydrate.

M.pt 198-200°

Anal. $C_{11}H_{11}FN_2O_5 . 0.5H_2O$ requires C,47.3; H,4.30; N,10.00

Found C,47.10; H,3.95; N,10.11.

'H NMR and UV data confirm the above structure.

**Example 2**

2′-Deoxy-5-ethynyl-2′fluorocytidine

To a solution of 2′-deoxy-2′-fluoro-5-iodouridine (500mg, 1.34mmol) in pyridine (8ml) at 0°C was added benzoyl chloride (0.33ml) with stirring. After 30 minutes the mixture was allowed to attain room temperature and stirring continued for a further 4 hours. The reaction was quenched with MeOH, evaporated, and co-evaporated with EtOH and toluene. The residue was chromatographed over flask $SiO_2$, eluting with toluene/EtOAc (6:1), then (4:1), to afford 2′-deoxy-3′,5′-di-O-benzoyl-2′-fluoro-5-iodouridine as a white solid.

A solution of 2′-deoxy-3′,5′-di-O-benzoyl-2′-fluoro-5-iodouridine (1.04g, 1.79mmol) in N-ethylmorpholine (16ml) was purged with oxygen-free dry $N_2$ . Cu(I)I (42.6mg), $PdCl_2(PPh_3)_2$ (45.5mg) and trimethylsilylacetylene (527mg) were added and the mixture stirred for one day at room temperature. CuI, $PdCl_2(PPh_3)_2$ and trimethylsilylacetylene were then added in the same amounts as before, and stirring continued for a further day. The solvent was evaporated, the residue dissolved in $CHCl_3$ and washed with 5% EDTA solution (twice) and water. The organic layer was dried ($Na_2SO_4$), evaporated and purified by flash chromatography over $SiO_2$, eluting with toluene/EtOAc (4:1). This provided 2′-deoxy-3′,5′-di-O-benzoyl-2′fluoro-5-(2-trimethylsilyl)-ethynyluridine.

To a solution of this compound (740mg, 1.34mmol) and 1,2,4-triazole (550mg) in pyridine at 0°C was added p-chlorophenyldichlorophosphate (0.59ml) dropwise with stirring. The mixture was allowed to attain room temperature, stirred for 16 hours, then quenched with water (30ml) and diluted with $CHCl_3$ (40ml). The organic layer was dried ($Na_2SO_4$) and evaporated in vacuo. The residue was dissolved in THF (15ml), and c.$NH_4OH$ (40ml) added. After stirring for 24 hours the solvent was evaporated and the residue treated with methanolic ammonia (saturated, 70ml), and $CH_2Cl_2$ (7ml). After a further 24 hours the solvent was removed in vacuo, and the residue chromatographed over flash $SiO_2$, eluting successively with $CHCl_3$/MeOH (10:1), (6:1), (4:1) and finally (3:1) to afford 2′-deoxy-5-ethynyl-2′-fluorocytidine. Recrystallisation from MeOH/$Et_2O$ gave pale yellow crystals.

M.pt 177-178° (dec)

Anal. $C_{11}H_{12}FN_3O_4$ requires C,49.07; H,4.49; N,15.61.

Found C,49.00; H,4.61; N,15.26.

'H NMR and UV data confirm the above structure.

**Example 3**

2′-Deoxy-2′-fluoro-5-prop-1-ynyluridine

To a solution of 2′-deoxy-3′,5′-di-O-benzoyl-2′-fluoro-5-iodouridine (560mg, 0.97mmol) in N-ethylmorpholine (15ml), previously purged with oxygen-free dry $N_2$, was added CuI (46mg), Pd $Cl_2(PPh_3)_2$ (50mg), and the solution saturated with propyne gas. After 24 hours, the solution was re-saturated with propyne gas and stirring continued for a further day. The solvent was then evaporated, the residue dissolved in $CHCl_3$ and washed with 5% EDTA solution (twice) and water. The organic fraction was dried ($Na_2SO_4$), evaporated, and chromatographed over flash $SiO_2$, eluting with toluene/EtOAc (6:1), then successively with (4:1), (3:1) and (1:1). This provided 2′-deoxy-3′,5′-di-O-benzoyl- 2′-fluoro-5-prop-1-ynyluridine.

A solution of this compound in MeOH was deprotected with sodium methoxide at room temperature over one day. The mixture was neutralised with Dowex ($H^+$) resin, filtered, the filtrate evaporated and chromatographed over flash $SiO_2$. Elution with $CHCl_3$, then successively with $CHCl_3$/MeOH(20:1), (10:1), (6:1), then finally (4:1) gave 2′-deoxy-2′-fluoro-5-prop-1-ynyluridine, which recrystallised from MeOH as a 0.2 hydrate.

M.pt 191°

Anal. $C_{12}H_{13}FN_2O_5.0.2H_2O$ requires C,50.11; H,4.70; N,9.74.

Found C,49.95; H,4.52; N,9.58.
'H NMR and UV data confirm the above structure.

**Example 4**

2'-Deoxy-2'-fluoro-5-prop-1-ynylcytidine

To a solution of 2'-deoxy-3',5'-di-O-benzoyl-5-prop-1-ynyluridine (630mg, 1.31mmol) and 1,2,4-triazole (540mg) in pyridine (9ml) at 0°C was added dropwise p-chlorophenyldichlorophosphate (0.58ml) with stirring. The reaction was stirred ovenight at room temperature, cooled to 0°C, and a further 270mg 1,2,4-triazole and 0.29ml p-chlorophenyldichlorophosphate added. After 3 hours at room temperature, $CHCl_3$ was added, the mixture washed with water, the organic fraction dried ($Na_2SO_4$) and evaporated to an oil. The oil was dissolved in THF (15ml), conc.ammonia (15ml) added, and the solution stirred overnight at room temperature. The solvent was then removed in vacuo and the residue chromatographed over flash $SiO_2$, eluting with $CHCl_3$/MeOH (20:1), then (6:1); the solvents containing 1% $Et_3N$. The fractions containing the product were combined, evaporated and taken up in a small volume of MeOH, from which 2'-deoxy-2'-fluoro-5-prop-1-ynylcytidine crystallised as a pale yellow solid.

M.pt 100°
Anal. $C_{12}H_{14}FN_3O_4$ . 1.37 $H_2O$ requires C,46.80; H,5.48; N,13.64.
Found C,46.54; H,5.22; N,13.50.
'H NMR, UV and mass spectra data also confirm this structure.

**Example 5**

Antiviral and Toxicity testing

Antiviral testing against VZV was determined by measuring the ability of the compound to reverse the cytophathic effect of VZV infection.

VZV is assayed in monolayers of either MRC5 cells (human embryonic lung) or Detroit 532 cells (human foreskin fibroblasts) in multiwell trays. Activity of compounds is determined in the plaque reduction assay, in which a cell monolayer is infected with a suspension of VZV. Plaque numbers of each concentration are expressed as percentage of the control and a dose-response curve is drawn. From this curve the 50% inhibitory concentration ($IC_{50}$) is estimated.

Cell toxicity is assessed in a cell growth inhibition assay. Subconfluent cultures of Vero cells grown on 96-well microtiter dishes are exposed to different dilutions of drug, and cell viability determined daily on replicate cultures using uptake of a tetrazolium dye (MTT). The concentration required for a 50% inhibition of cell vaibility at 96 hours is termed $CCID_{50}$.

The results are shown in the following Table.

Results

| Compound | VZV 1C50/μM | Toxicity CCID50(Vero)/μM |
|---|---|---|
| 2'-Deoxy-5-ethynyl-2'-fluorouridine | 2.16 | 156 |
| 2'-Deoxy-5-ethynyl-2'-fluorocytidine | 5.3 | >500 |
| 2'-Deoxy-2'-fluoro-5-prop-1-ynyluridine | 2.6 | >500 |
| 2'-Deoxy-2'-fluoro-5-prop-1-ynylcytidine | 2.3 | >500 |

## Claims

1. A compound of the formula (I)

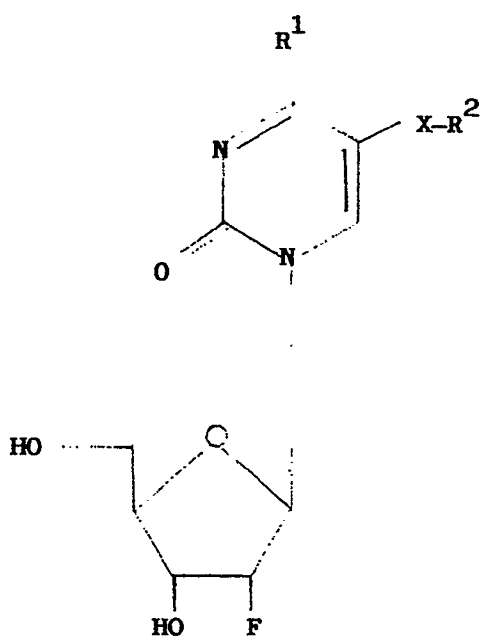

(I)

wherein $R^1$ represents a hydroxy or amino group;
X represents a $C_{2-4}$ alkynylene group, a $C_{2-4}$ alkenylene group optionally substituted by one or more halogen radicals or a $C_{1-4}$ alkylene group optionally substituted by one or more halogen radicals;
and $R^2$ represents a hydrogen atom, a $C_{1-4}$ alkyl group, a $C_{3-4}$ branched alkyl, a $C_{3-4}$ cycloalkyl group or a $C_{3-6}$ cycloalky-$C_{1-6}$ alkyl group; with the proviso that the compound of formula (I) is not 5-vinyl-2'-fluoro-2'deoxyuridine;
or a physiologically acceptable salt, ester or salt of ester thereof.

2. A compound as claimed in Claim 1, wherein $R^1$ and $R^2$ are as hereinbefore defined and X represents a $C_1$ alkynyline group.

3. A compound as claimed in Claim 1, wherein $R^1$ and X are as hereinbefore defined and $R^2$ represents a hydroxy or amino group.

4. 2'-deoxy-2'-fluoro-5-prop-1-ynyluridine or a physiologically acceptable salt, ester or salt of ester thereof.

5. 2'-deoxy-5-ethynyl-2'-fluorocytidine or a physiologically acceptable salt, ester or salt of ester thereof.

6. 2'-deoxy-2'-fluoro-5-prop-1-ynylcytidine or a physiologically acceptable salt, ester or salt of ester thereof.

7. 2'-deoxy-5-ethynyl-2'-fluorouridine or a physiologically acceptable salt, ester or salt of ester thereof.

8. A process for the preparation of a compound as defined in any one of claims 1 to 7 or a physiologically acceptable salt, ester or salt of ester thereof which comprises:
   (a) condensing a compound of formula (II)

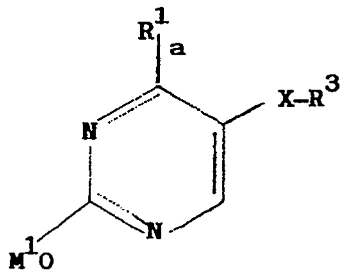

(II)

(wherein $R^3$ is a protecting group which can be converted to H (eg. $SiR^4_3$ wherein $R^4$ is a $C_{1-4}$ alkyl group) or is defined as for $R^2$, X is as defined above (p.3), $R^1_a$ represents a protected hydroxy or amino group and $M^1$ represents a hydroxy protecting group) with a compound of formula (III)

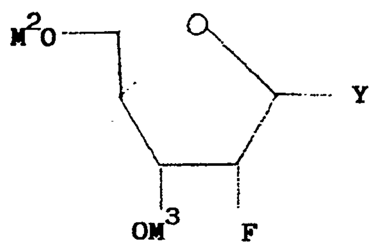

(III)

(wherein Y represents a halogen atom, acyl group or alkyl group (eg. $-CO-R^5$ wherein $R^5$ is $C_{1-4}$ alkyl) and $M^2$ and $M^3$ each represent a hydroxy-protecting group; or

(b) reacting a compound of formula (IV)

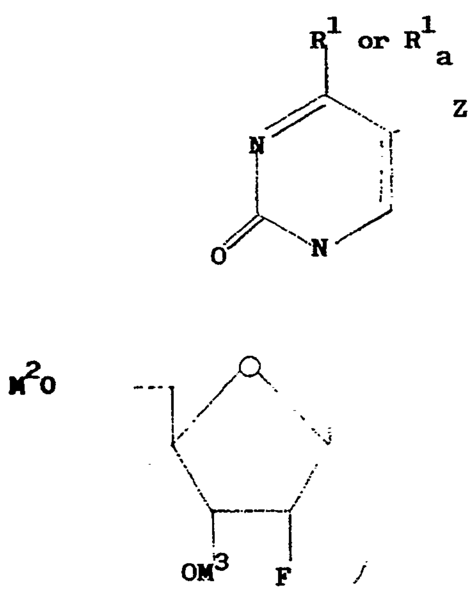

(IV)

wherein $R^1$ and $R^1_a$, $M^2$ and $M^3$ are as defined above and Z is a leaving group, with a compound capable of providing the necessary grouping of formula $-X-R^3$ (in which X is ethynylene and $R^3$ is as defined above); or

(c) reacting a compound of formula (V)

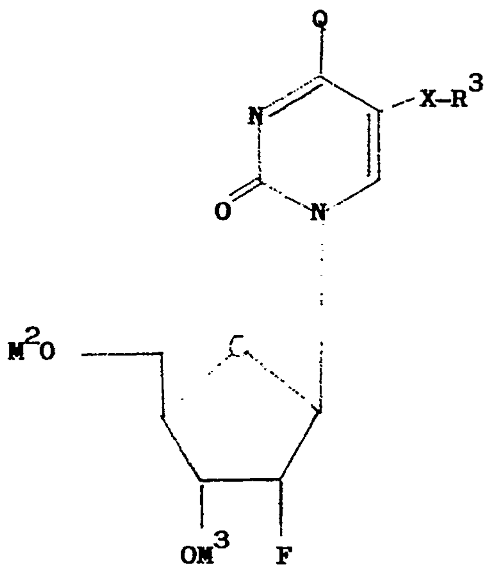

(V)

(wherein Q is an appropriate leaving group and X,$R^3$,$M^2$ and $M^3$ are as defined above) with an agent serving to replace the group Q with an amino group to form a compound of formula (I) in which $R^1$ is amino and $R^3$ is as described above; or

(d) decarboxylative halogenation (e.g. using an N-halosuccinimide) of a compound of formula (VI) below

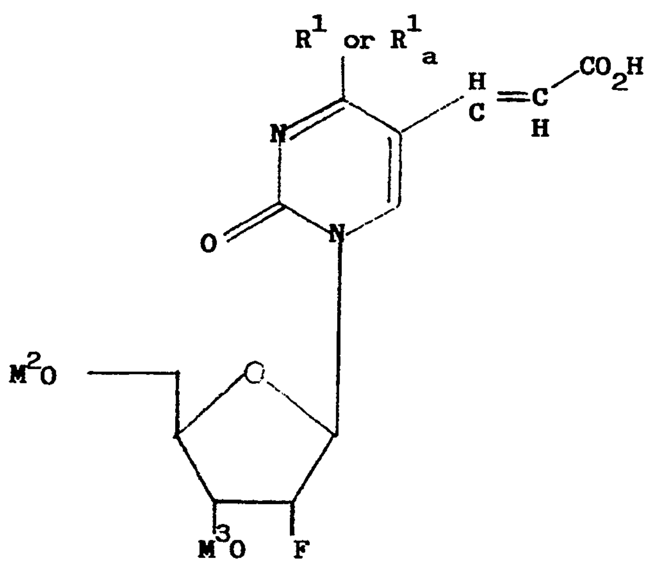

(VI)

wherein $R^1$,$R^1_a$, $M^2$ and $M^3$ are as defined above to form a compound of formula (I) in which X is ethenylene substutited with a halogen atom and $R^2$ is a hydrogen; or

(e) halogenating a compound of formula (VII) below

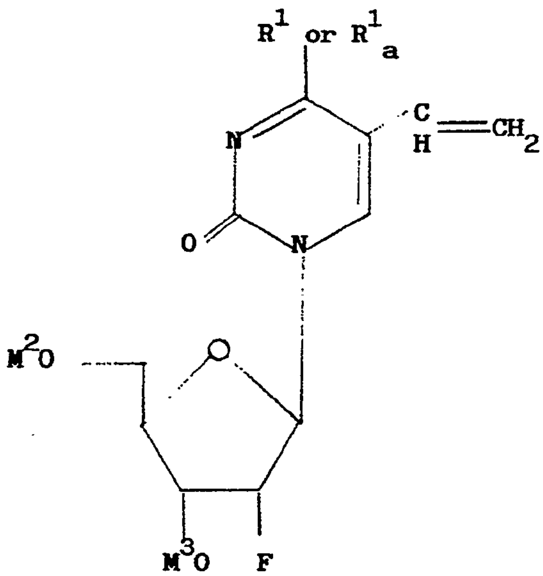

(VII)

wherein $R^1$, $R^1_a$, $M^2$ and $M^3$ are as defined above to form a compound of formula (I), in which X is ethenylene substituted with a halogen atom and $R^2$ is a hydrogen atom; or

(f) reacting a compound of formula (VIII)

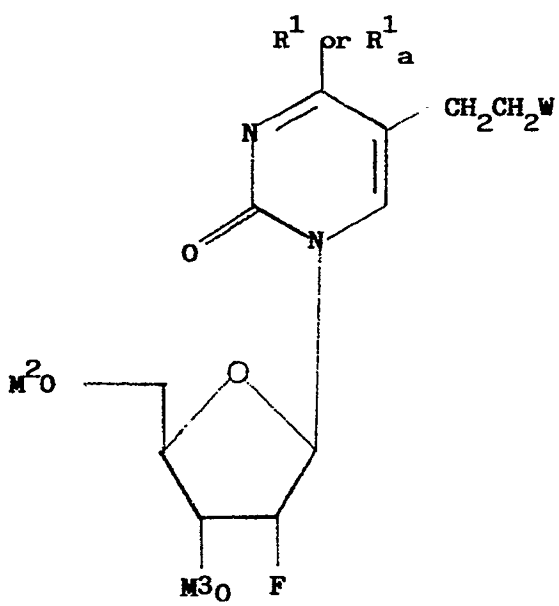

(VIII)

(wherein W is a leaving group) under conditions serving to replace the said leaving group by a halogen atom to form a compound of formula I wherein X is a halogen-substituted alkylene group and $R^2$ is hydrogen; or

(g) reacting a compound of formula (IX)

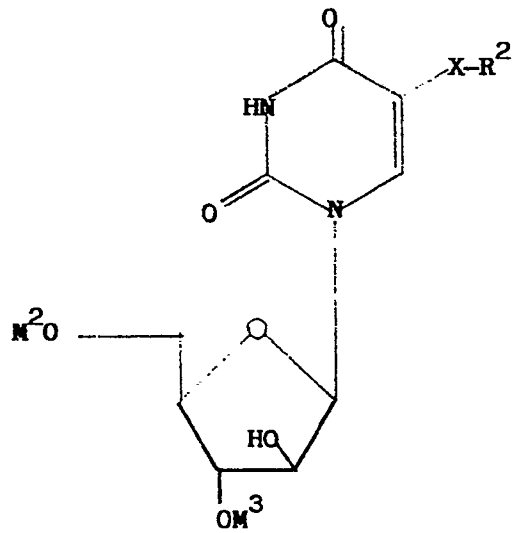

(IX)

wherein X, $R^2$, $M^2$ and $M^3$ are as defined above) with a dialkylamino sulphur trifluoride e.g. diethylamino sulphur trifluoride or other suitable derivative of $SF_4$; or

(h) reacting a compound of formula (X)

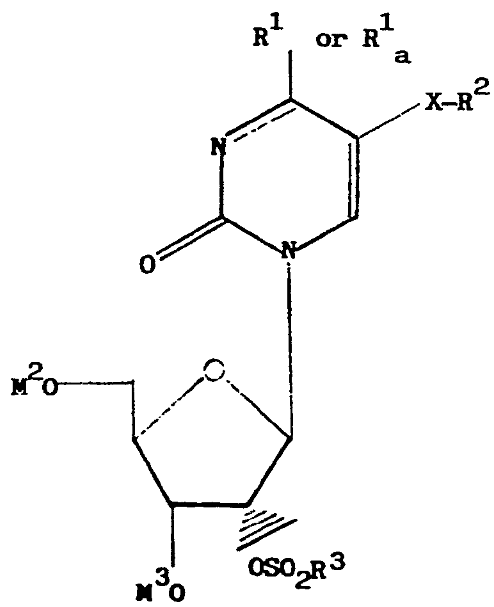

(X)

wherein X, $R^2$, $R^3$, $M^2$ and $M^3$ are as defined above) with a source of fluoride ions such as potassium fluoride or tetrabutylammonium fluoride advantageously in a suitable solvent such as dimethylformamide or tetrahydrofuran;

and optionally thereafter or simultaneously therewith, performing either or both of the following, in any desired order:

(i) removing any remaining protecting groups

(ii) where the resulting compound is a compound of formula (I), converting it into a physiologically acceptable salt, ester or salt of ester thereof or, where the resulting compound is a physiologically acceptable salt, ester or salt of ester, converting it into a different physiologically acceptable salt, ester or salt of ester or a compound of formula (I).

9. A pharmaceutical formulation which comprises a compound of formula (I) or physiologically acceptable salt, ester or salt of ester thereof according to any of of claims 1 to 7 in association with a pharmaceutically acceptable carrier.

10. A compound as claimed in any of claims 1 to 7 for use in therapy.

11. Use of a compound or a physiologically acceptable salt, ester or salt of ester thereof as claimed in any of claims 1 to 7 in the manufacture of a medicament for the treatment or prophylaxis of herpes virus infections.

12. Use of a compound or a phusiologically acceptable salt, ester or salt of ester thereof as claimed in any of claims 1 to 7 in the manufacture of a medicament for the treatment or prophylaxis of HSV-1.

13. Use of a compound or a pharmaceutically acceptable ester or salt thereof as claimed in any of claims 1 to 7 in the manufacture of a medicament for the treatment or prophylaxis of VZV.

**Claims for the following Contracting States: ES, GR**

1. A process for producing a compound of formula (I)

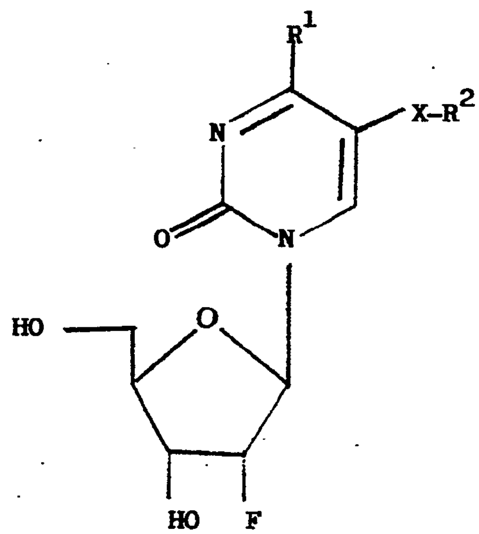

(I)

wherein $R^1$ represents a hydroxy or amino group;
X represents a $C_{2-4}$ alkynylene group; a $C_{2-4}$ alkenylene group optionally substituted by one or more halogen radicals or a $C_{1-4}$ alkylene group optionally substituted by one or more halogen radicals;
and $R^2$ represents a hydrogen atom, a $C_{1-4}$ alkyl group, a $C_{3-4}$ branched alkyl group, a $C_{3-4}$ cycloalkyl group or a $C_{3-6}$ cycloalkyl-$C_{1-6}$alkyl group; with the proviso that the compound of formula (I) is not 5-vinyl-2′-fluoro-2′-deoxyuridine;
or a physiologically acceptable salt, ester or salt of ester thereof;
which comprises
(a) condensing a compound of formula (II)

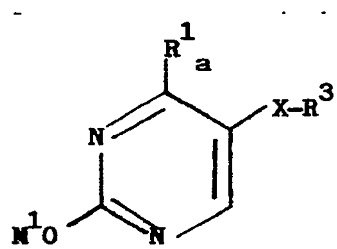

(II)

(wherein $R^3$ is a protecting group which can be converted to H (eg. $SiR^4_3$ wherein $R^4$ is a $C_{1-4}$ alkyl group) or is defined as for $R^2$, X is as hereinbefore defined, $R^1_a$ represents a protected hydroxy or amino group and $M^1$ represents a hydroxy protecting group) with a compound of formula (III)

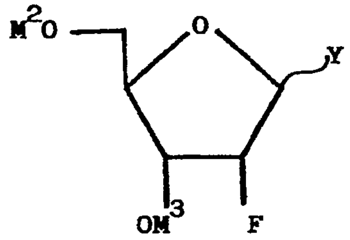

(III)

(wherein Y represents a halogen atom, acyl group or alkyl group (eg -CO-$R^5$ wherein $R^5$ is $C_{1-4}$ alkyl) and $M^2$ and $M^3$ each represent a hydroxy-protecting group; or

(b) reacting a compound of formula (IV)

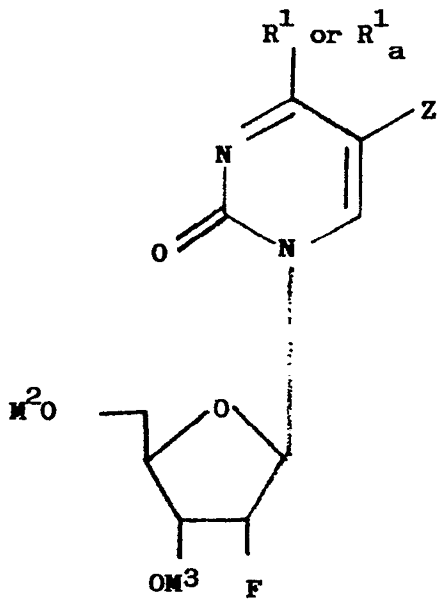

(IV)

wherein $R^1$ and $R^1_a$, $M^2$ and $M^3$ are as defined above and Z is a leaving group, with a compound capable of providing the necessary grouping of formula -X-$R^3$ (in which X is ethynylene and $R^3$ is as defined above); or

(c) reacting a compound of formula (V)

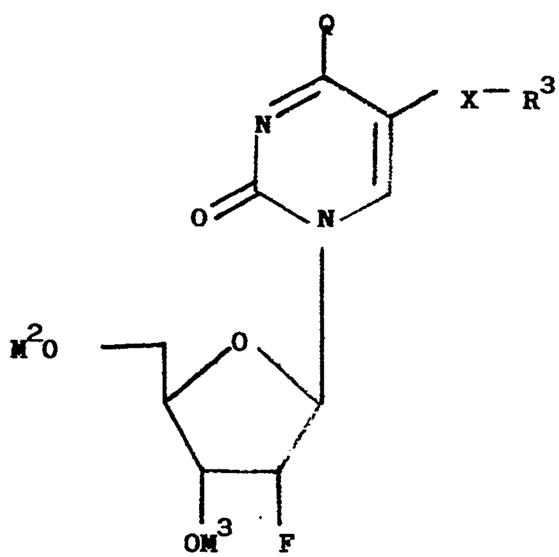

(V)

(wherein Q is an appropriate leaving group and X,$R^3$,$M^2$ and $M^3$ are as defined above) with an agent serving to replace the group Q with an amino group to form a compound of formula (I) in which $R^1$ is amino and $R^3$ is as described above; or
(d) a decarboxylative halogenation (e.g. using an N-halosuccinimide)

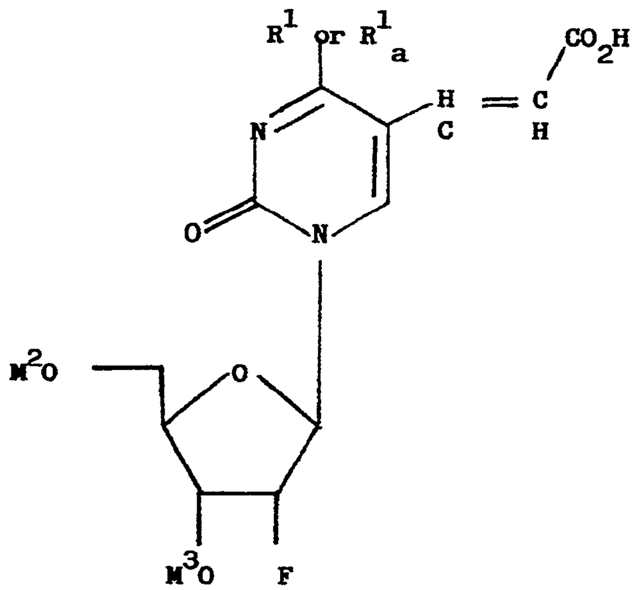

(VI)

wherein $R^1$, $R^1_a$, $M^2$ and $M^3$ are as defined above to form a compound of formula (I) in which X is ethenylene substituted with a halogen atom and $R^2$ is hydrogen; or
(e) halogenating a compound of formula (VII) below

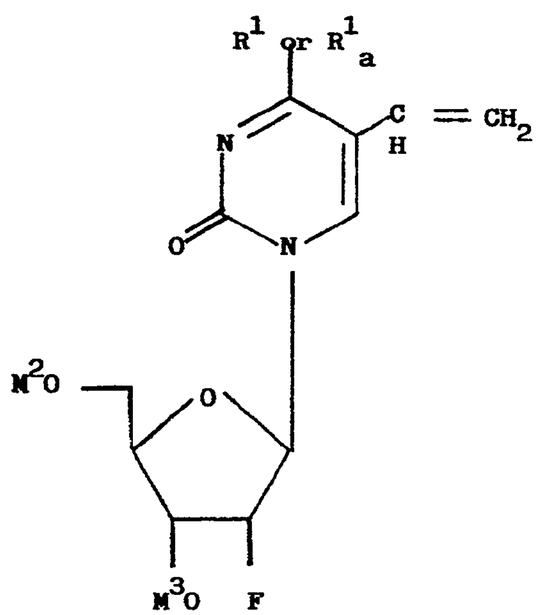

(VII)

wherein $R^1$, $R^1_a$ , $M^2$ and $M^3$ are as defined above to form a compound of a formula (I), in which X is ethenylene substituted with a halogen atom and $R^2$ is a hydrogen atom; or
(f) reacting a compound of formula (VIII)

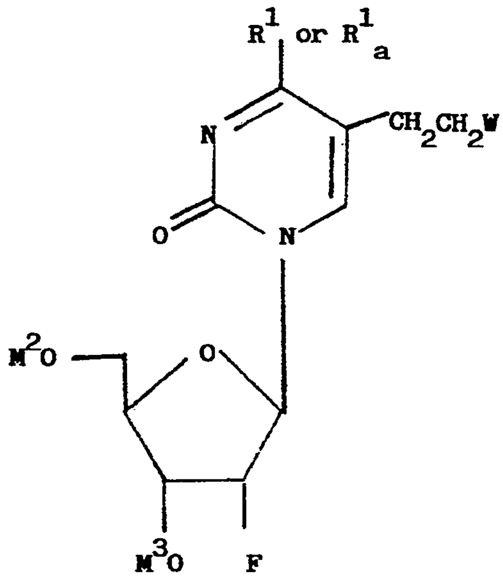

(VIII)

(wherein W is a leaving group) under conditions serving to replace the said leaving group by a halogen atom to form a compound of formula I wherein X is a halogen-substituted alkyline group and $R^2$ is hydrogen;

(g) reacting a compound of formula (IX)

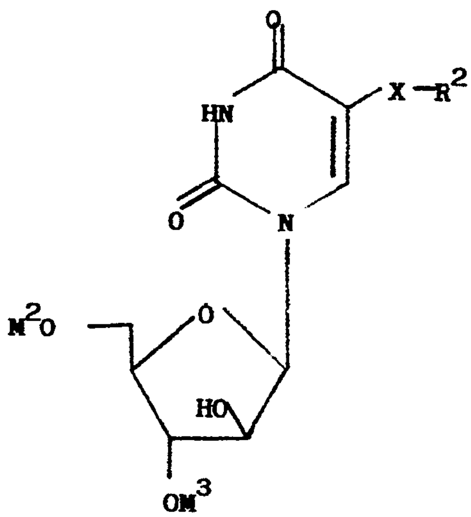

(IX)

(wherein X, $R^2$, $M^2$ and $M^3$ are as defined above) with a dialkylamino sulphur trifluoride e.g. diethylamino sulphur trifluoride or other suitable derivative of $SF_4$;

(h) reacting a compound of formula (X)

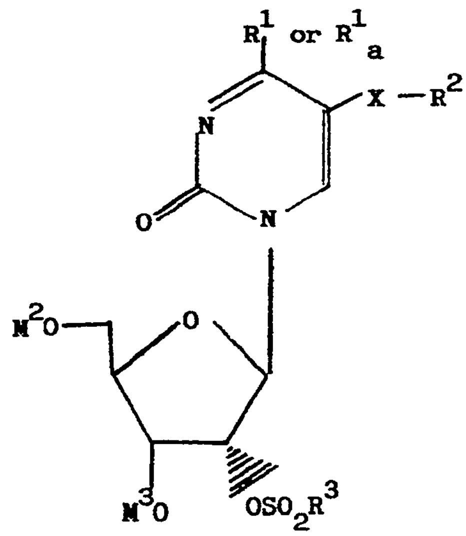

(X)

(wherein X, $R^2$, $R^3$, $M^2$ and $M^3$ are as defined above) with a fluoride source such as potassium fluoride or tetrabutylammonium fluoride in a suitable solvent such as dimethylformamide or tetrahydrofuran; and optionally thereafter or simultaneously therewith, performing either or both of the following, in any desired order:

(i) removing any remaining protecting groups

(ii) where the resulting compound is a compound of formula (I), converting it into a physiologically acceptable salt, ester or salt of ester thereof or, where the resulting compound is a physiologically acceptable salt or ester, converting it into a different physiologically acceptable salt , ester or salt of ester or a compound of formula (I).

**2.** A process as claimed in Claim 1, wherein $R^1$ and $R^2$ are as hereinbefore defined and X represents a $C_1$ alkynyline group.

**3.** A process as claimed in Claim 1, wherein $R^1$ and X are as hereinbefore defined and $R^2$ represents a hydroxy or amino group.

**4.** A process as claimed in any of claims 1-3 for producing 2′-deoxy-2′-fluoro-5-prop-1-ynyluridine or a physiologically acceptable salt, ester or salt of ester thereof.

**5.** A process as claimed in any of claims 1-3 for producing 2′-deoxy-5-ethynyl-2′-fluorocytidine or a physiologically acceptable salt, ester or salt of ester thereof.

**6.** A process as claimed in any of claims 1-3 for producing 2′-deoxy-2′-fluoro-5-prop-1-ynylcytidine or a physiologically acceptable salt, ester or salt of ester thereof.

**7.** A process as claimed in any of claims 1-3 for producing 2′-deoxy-5-ethynyl-2′-fluorouridine or a physiologically acceptable salt, ester or salt of ester thereof.

**8.** A method of producing a pharmaceutical formulation comprising mixing a compound of formula (1) as claimed in claim 1 with a physiologically acceptable carrier therefor.